# EUROPÄISCHE PATENTSCHRIFT

(11) **EP 0 702 963 B1**
(45) Veröffentlichungstag und Bekanntmachung des Hinweises auf die Patenterteilung: **20.01.1999**
(21) Anmeldenummer: 95106268.6
(22) Anmeldetag: 26.04.1995
(51) Int. Cl.: A61L 2/18, A61L 2/04

(54) **Verfahren zum maschinellen Reinigen und Desinfizieren von Apparaten und Instrumenten**
Method for machine-cleaning and disinfecting of apparatus and instruments
Procédé de nettoyage et de désinfection d'appareils et d'instruments à l'aide d'une machine

(30) Priorität: 26.09.1994 DE 4434308
(43) Veröffentlichungstag der Anmeldung: 27.03.1996
(73) Patentinhaber: CHEMISCHE FABRIK DR. WEIGERT (GMBH & CO.), 20539 Hamburg (DE)
(72) Erfinder: Staffeldt, Jürgen, Dr., D-21423 Winsen, Luhe (DE)
(74) Vertreter: Glawe, Delfs, Moll & Partner

(56) Entgegenhaltungen:
- DE-A- 2 749 448
- DE-A- 2 852 072
- DE-A- 3 601 744

## Beschreibung

Die Erfindung betrifft ein Verfahren zum maschinellen Reinigen und thermischen Desinfizieren von medizinischen und biotechnologischen Apparaten und Instrumenten in einer Eintankmaschine.

Für die Reinigung und Desinfektion von medizinischen oder chirurgischen sowie biotechnologischen Instrumenten und Apparaturen werden Spezialspülmaschinen eingesetzt; die Desinfektion erfolgt thermisch. Als Spülmaschinen werden hier im wesentlichen zwei Arten von Maschinen eingesetzt, und zwar einerseits sog. Eintankmaschinen mit Frischwasserwechsel, z.B. gemäß DE-A-3 601 777 und DE-A-3 327 466, und andererseits sog. Taktstraßen, in denen die Instrumente nacheinander verschiedene Reinigungs- und Desinfektionszonen durchlaufen.

Der Programmablauf bei den Eintankmaschinen ist typischerweise wie folgt: Kalt- oder Warmwasser läuft in die Maschine ein und wird für eine Vorreingiung verwendet. Danach erfolgt entweder mit erneutem Frischwasser oder mit dem bereits eingelaufenen Kalt- oder Warmwasser eine alkalische Reinigung durch Hinzudosieren eines alkalischen Reinigungsmittels und Aufheizen der Reinigerflotte auf Temperaturen von 45-60°C.

Nach einer Temperaturhaltezeit, in welcher die Instrumente gereinigt werden, erfolgt das Abpumpen der Reinigerflotte; anschließend erfolgt eine Neutralisation mit einem sauren Reagens sowie eine Nachspülung mit Wasser in mehreren Nachspülgängen. Die letzte Nachspülung wird hier optimalerweise mit vollentsalztem Wasser vorgenommen, und dieses letzte Nachspülwasser wird auf Thermodesinfektionstemperaturen von 85-93°C mit entsprechender Temperaturhaltezeit erhitzt.

Speziell bei hartnäckigeren Blutrückständen und -verkrustungen wird im allgemeinen mit einer schwach temperierten sauren Vorreinigerflotte ein besseres Reinigungsergebnis erzielt. Ein saurer Reiniger hat jedoch den Nachteil, daß Abflußrohre von der Spülmaschine säurebeständig sein müssen. Zur Komplettierung eines erfolgreichen Reinigungsergebnisses muß dennoch ein alkalischer Reinigungsgang gefahren werden, was bisher nur in getrennten Schritten möglich war.

Da eine Kombination einer sauren Reinigung mit einer anschließenden pH-neutralen oder alkalischen Reinigung verbesserte Reinigungsergebnisse liefert, besteht ein Bedürfnis an Verfahren für Eintankmaschinen, in denen diese Reinigungsschritte ohne die Entstehung von saurem Abwasser und demzufolge mit dem weiteren Vorteil verkürzter Programmzeiten sowie einer Wasserersparnis durchgeführt werden können.

Dieses Ziel läßt sich erfindungsgemäß dadurch erreichen, daß man in Eintankmaschinen der handelsüblichen Art ein Verfahren einsetzt, gemäß dem man die Apparate und Instrumente mit einer sauren bzw. sauer eingestellten Reinigerflotte bei Temperaturen bis zu 55°C behandelt, der Reinigerflotte in Gegenwart der Apparate und Instrumente ein mit dieser verträgliches basisches Reinigungs- und Neutralisationsmittel unter Einstellung eines pH-Wertes von mindestens 6,5 zusetzt und die Apparate und Instrumente, gegebenenfalls unter weiterem Erhitzen der Reinigerflotte, weiterreinigt, und daß man die alkalische Reinigerlösung, gegebenenfalls nach Neutralisation derselben, entfernt sowie die Apparate und Instrumente mindestens einmal mit Frischwasser und zum Abschluß mit entmineralisiertem Frischwasser oder Weichwasser nachspült, wobei die Nachspülung mit dem entmineralisierten Frischwasser oder Weichwasser unter thermischen Desinfektionsbedingungen erfolgt.

Gemäß dem Verfahren der Erfindung wird somit eine Eintankmaschine der üblichen Art mit Frischwasserwechsel mit Kaltwasser befüllt. Dem kalten Wasser wird ein sauer Reiniger zudosiert; das sauer eingestellte Reinigungsmedium wird anschließend für die Reinigung in einem relativ niedrigen Temperaturbereich benutzt. Damit lassen sich Beschädigungen an Instrumenten, insbesondere durch Korrosion, ausschalten. Mit saurer Reinigerflotte besser entfernbare Rückstände an Apparaten und Instrumenten werden hierbei schon entfernt.

Nach einer gewissen Einwirk- bzw. Reinigungszeit, wobei die dabei verwendete kalte, saure Reinigungslösung auch allmählich auf Temperaturen bis zu 55°C, insbesondere bis zu 40°C, erwärmt werden kann, erfolgt die Zudosierung eines alkalischen Reinigungs- und Neutralisationsmittels zur Erzeugung der für die weitere Reinigung erforderlichen alkalischen Einstellung der Reinigungslösung. Der Reiniger muß dabei so beschaffen sein, daß er beim Zudosieren in die saure Reinigungslösung nicht zu Ausflockungen oder Ausfällungen führt. Dabei wird neutralisiert, bis der pH-Wert mindestens 6,5 oder bis zu 7,5 beträgt, oder es wird insbesondere überneutralisiert, bis der pH-Wert der Reinigerlösung im Bereich von 9 bis 12 liegt. Anschließend erfolgt eine weitere Reinigung mit dieser alkalisierten Reinigerflotte. Anschließend schließen sich die üblichen Neutralisations- und Nachspülgänge an, wobei vorzugsweise in die alkalisierte Reinigungslösung das Neutralisationsmittel hineindosiert wird, so daß ein pH-neutrales Abwasser die Spülmaschine verläßt.

Unter dem erfindungsgemäß bei der Nachspülung zu verwendenden Weichwasser ist natürlich vorkommendes Wasser mit einem niedrigen Gehalt an Mineralstoffen zu verstehen. Unter entmineralisiertem Frischwasser ist Wasser zu verstehen, dem auf üblichen Wege, z.B. durch Ionenaustausch oder Umkehrosmose, die in ihm enthaltenen Mineralstoffe vollständig oder teilweise entzogen worden sind.

Das Verfahren der Erfindung hat den besonderen Vorteil, daß die Reinigung der zu behandelnden Apparate und Instrumente einmal im sauren und zum anderen im neutralen bzw. alkalischen Milieu erfolgt, so daß auch solche Verunreinigungen wirksam entfernt werden, die sich nur im Sauren oder nur im Neutralen bis Alkalischen besonders gründlich entfernen lassen, und trotzdem auch das Abwasser nicht mit sauren oder alkalischen Reinigungsmittellösungen belastet wird.

Gemäß einer bevorzugten Ausführungsform weist die Reinigerflotte einen pH-Wert im Bereich von 1 bis 4 auf; die Behandlung mit der sauren Reinigerflotte erfolgt vorzugsweise im Temperaturbereich zwischen 10 und 30°C, insbesondere bei Umgebungstemperatur.

Gemäß einer weiteren vorteilhaften Ausführungsform der Erfindung weist die Reinigerflotte nach der Neutralisation bzw. der Alkalisierung einen pH-Wert im Bereich von 7 bis 12, insbesondere von 9 bis 12, auf.

Nach Beendigung des sauren und alkalischen Reinigungsschrittes sowie der erfolgten Neutralisation der Reinigerflotte erfolgt die Neubschickung der Spülmaschine mit Frischwasser zur Nachspülung. In einem zweiten Nachspülschritt, der gleichzeitig der letzte Spülschritt in der Maschine ist, erfolgt eine Aufheizung dieses letzten Spülwassers, vorzugsweise entmineralisiertem Wasser, bis zu Thermodesinfektionstemperaturen. Die Thermodesinfektionstemperaturen richten sich nach national unterschiedlichen Hygienevorschriften und können beispielsweise 5 min bei 85°C, 3 min bei 93°C oder 10 min bei 93°C sein.

Anschließend erfolgen die üblichen thermischen Trocknungsstufen, womit das Programm abgeschlossen wird.

Nachfolgend wird ein Ausführungsbeispiel für die Durchführung des Verfahrens der Erfindung gegeben.

| Programmschritt/Funktion | Beispiel |
|---|---|
| Wassereinlauf, enthärtet oder demineralisiert | 10 l kalt = 10-20°C |
| Dosierung eines sauren Reinigungsmittels, 10-20ml auf 10 l Wasservolumen | pH-Wert 2,5-3,5 |
| Saure Reinigungsphase | Wassereinlauftemperatur ohne Einschalten der Heizung oder Aufheizen der sauren Reinigungslösung auf bis zu 55°C; Zeit: 0,5-5 min |
| Zudosierung des alkalischen Reinigers, 30-40 ml auf 10 l Wasser | pH-Wert > 11 |
| | Erfolgt die saure Reinigung bei Wassereinlauftemperaturen, wird der alkalische Reiniger nach Abschluß der sauren Reinigung zudosiert; erfolgt eine Aufheizung der sauren Reinigungslösung, wird die Zudosierung des alkalischen Reinigers vorgenommen, wenn die saure Reinigungslösung eine Temperatur von etwa 40°C erreicht hat. |
| Weiteres Aufheizen bis zur alkalischen Reinigungstemperatur | 45-70°C |
| Neutralisation, Dosierung eines sauren Neutralisationsmittels, 10-20 ml auf 10 l Wasser | pH-Wert 6-8 |
| Zwischenspülung mit Frischwasser, 10 l | |
| Letzter Spülgang sowie Thermodesinfektionsschritt, Wassereinlauf enthärtet oder demineralisiert, 10 l | 3 min, 93°C |

### Thermische Trocknung

Typische Beispiele für in dem Verfahren der Erfindung einzusetzende saure Reiniger sind organische Monocarbonsäuren wie Ameisensäure oder Essigsäure oder Hydroxycarbonsäuren wie Zitronensäure oder Äpfelsäure. Auch anorganische Säuren wie Phosphorsäure, Schwefelsäure, Salpetersäure oder Amidosulfonsäure sind möglich, weiterhin können auch Persäuren, z. B. Peressigsäure, eingesetzt werden.

Der saure Reiniger kann außer den oben beschriebenen Säuren Hilfsstoffe wie Dispergatoren, Netzmittel, korrosionsverhindernde Zusätze oder dergleichen enthalten.

Der in dem Verfahren der Erfindung einsetzbare alkalische Reiniger kann z. B. Kaliumhydroxid, Natriumhydroxid, Nitrilotrisessigsäure, EDTA (Ethylendiamintetraessigsäure), Polyphosphat, Härtedispergatoren, Tenside sowie korrosionsverringernde Zusätze, z. B. Aminderivate wie Triethanolamin enthalten. Die vorerwähnten Inhaltsstoffe sind an sich nicht neu und sind dem Reinigungsfachmann auf dem hier angesprochenen Gebiet bestens bekannt.

Eine besonders bevorzugte Rezeptur für den sauren und den alkalischen Reiniger zur Verwendung in dem Verfahren der Erfindung wird im folgenden wiedergegeben.
- Saurer Reiniger:: 62 % Phosphorsäure
2 % Zitronensäure
Rest: vollentsalztes Wasser
- Alkalischer Reiniger:: 14 % Natriumhydroxid
16 % Nitrilotriessigsäure
1,5 % Tensid (Natriumalkylaminodipropionat)
Rest: vollentsalztes Wasser

Die vorgenannten Prozentzahlen beziehen sich auf Gew.-%.

### Beispiel 2:

Es wurde eine handelsübliche Eintankmaschine vom Typ Miele G 7735 OP zur Instrumentenaufbereitung mit Mikroprozessorsteuerung verwendet. Der Programmablauf war wie folgt:

In die Spülmaschine wurden 10 l kaltes enthärtetes Wasser eingegeben und mit 10 ml eines sauren Reinigers mit 39 Gew.-% Zitronensäure und 1 Gew.-% Äpfelsäure versetzt. Die resultierende saure Reinigungslösung mit pH 2,9 wurde mittels Umwälzpumpe der Spülmaschine 2 min lang für eine saure Reinigung chirurgischer Instrumente verwendet. Anschließend wurde die saure Reinigungslösung durch automatisches Einschalten der Heizung auf 40°C aufgeheizt. Hierfür wurde eine Zeit von 4 min benötigt. Anschließend erfolgte eine Zudosierung von 30 ml eines alkalischen Reinigers. Der alkalische Reiniger enthielt 14 % Natriumhydroxid, 16 % NTA sowie 1,5 % Natriumalkylaminodipropionat (handelsüblich). Nach Zudosierung des alkalischen Reinigers wies die Flotte einen pH-Wert von 11,6 auf. Ausflokkungen in der nun alkalisierten Reinigerflotte wurden nicht beobachtet. Während der Zudosierung des alkalischen Reinigers und nach Beendigung der Dosierung wurde bis auf 60°C aufgeheizt und diese Temperatur für die weitere Reinigung 5 min gehalten. Dann wurden 20 ml eines Neutralisationsmittels mit 39 Gew.-% Zitronensäure und 1 Gew.-% Äpfelsäure zudosiert, wobei sich ein pH-Wert von 6,0 einstellte.

Diese neutralisierte Reinigerlösung wurde aus der Maschine abgepumpt und es erfolgten dann die üblichen bekannten Programmabläufe des Nachspülens und der Thermodesinfektion mit dem letzten Nachspülwasser und des Trocknens.

## Patentansprüche

1. Verfahren zum maschinellen Reinigen und thermischen Desinfizieren von medizinischen und biotechnologischen Apparaten und Instrumenten in einer Eintankmaschine, durch gekennzeichnet, daß man die Apparate und Instrumente mit einer sauren bzw. sauer eingestellten Reinigerflotte bei Temperaturen bis zu 55°C behandelt, der Reinigerflotte in Gegenwart der Apparate und Instrumente ein mit dieser verträgliches basisches Reinigungs- und Neutralisationsmittel unter Einstellung eines pH-Wertes von mindestens 6,5 zusetzt und die Apparate und Instrumente, gegebenenfalls unter weiterem Erhitzen der Reinigerflotte, weiterreinigt, und daß man die alkalische Reinigerlösung, gegebenenfalls nach Neutralisation derselben, entfernt sowie die Apparate und Instrumente mindestens einmal mit Frischwasser und zum Abschluß mit entmineralisiertem Frischwasser oder Weichwasser nachspült, wobei die Nachspülung mit dem entmineralisierten Frischwasser oder Weichwasser unter thermischen Desinfektionsbedingungen erfolgt.

2. Verfahren nach Anspruch 1, dadurch gekennzeichnet, daß die Reinigerflotte einen pH-Wert im Bereich von 1 bis 4 aufweist.

3. Verfahren nach Anspruch 1 oder 2, dadurch gekennzeichnet, daß die Behandlung mit der sauren Reinigerflotte im Temperaturbereich zwischen 10 und 30°C, insbesondere bei Umgebungstemperatur erfolgt.

4. Verfahren nach mindestens einem der Ansprüche 1 bis 3, dadurch gekennzeichnet, daß die Reinigerflotte nach der Neutralisation bzw. der Alkalisierung einen pH-Wert im Bereich von 7 bis 12, insbesondere von 9 bis 12, aufweist.

5. Verfahren nach mindestens einem der Ansprüche 1 bis 4, dadurch gekennzeichnet, daß die thermische Desinfektion bei Temperaturen über 84°C, insbesondere über 90°C erfolgt.

6. Verfahren nach Anspruch 5, dadurch gekennzeichnet, daß die thermische Desinfektion über mindestens 3 min, insbesondere mindestens 10 min erfolgt.

## Claims

1. A method for the mechanical cleaning and thermal disinfection of medical and biotechnological apparatus and instruments in a single-tank machine, characterised in that the apparatus and instruments are treated with an acid or acidified cleansing liquor at temperatures of up to 55°C, in the presence of the apparatus and instruments a compatible basic cleansing and neutralising agent is added to the cleansing liquor, the pH being adjusted to a value of at least 6.5 and the apparatus and instruments, optionally with further heating of the cleansing liquor, are cleansed further, and in that the alkaline cleansing liquor is removed, optionally after neutralisation thereof, and the apparatus and instruments are rinsed at least once with fresh water and then, finally, with demineralised fresh water or soft water, the rerinsing with the demineralised fresh water or soft water taking place under thermal disinfecting conditions.

2. A method according to Claim 1, characterised in that the cleansing liquor has a pH value in the range of from 1 to 4.

3. A method according to Claim 1 or 2, characterised in that the treatment with the acid cleansing liquor takes place in the temperature range of between 10 and 30°C, in particular at ambient temperature.

4. A method according to at least one of Claims 1 to 3, characterised in that, after the neutralisation and/or basification, the cleansing liquor has a pH value in the range of from 7 to 12, in particular from 9 to 12.

5. A method according to at least one of Claims 1 to 4, characterised in that the thermal disinfection takes place at temperatures above 84°C, in particular above 90°C.

6. A method according to Claim 5, characterised in that the thermal disinfection takes place over at least 3 min., in particular at least 10 min.

## Revendications

1. Procédé pour le nettoyage mécanique et la désinfection thermique d'appareils et instruments médicaux et biotechnologiques dans une machine monobac, caractérisé en ce que l'on traite les appareils et les instruments avec un bain d'agent nettoyant acide ou à réglage acide à des températures atteignant 55°C, en ce qu'on ajoute au bain d'agent nettoyant contenant les appareils et les instruments un agent basique neutralisant et nettoyant compatible avec le bain en prévoyant une valeur de pH d'au moins 6,5 et on poursuit le nettoyage des appareils et des instruments tout en continuant éventuellement à chauffer le bain d'agent nettoyant, en ce qu'on élimine la solution alcaline nettoyante après l'avoir éventuellement neutralisée et en ce que l'on rince au moins une fois les appareils et les instruments à l'eau claire et pour finir à l'eau fraîche déminéralisée ou à l'eau adoucie, le rinçage s'effectuant à l'eau fraîche déminéralisée ou adoucie dans des conditions thermiques de désinfection.

2. Procédé selon la revendication 1, caractérisé en ce que le bain d'agent nettoyant présente une valeur de pH comprise entre 1 et 4.

3. Procédé selon la revendication 1 ou 2, caractérisé en ce que le traitement au bain nettoyant acide s'effectue à des températures comprises entre 10° et 30°C, en particulier à la température ambiante.

4. Procédé selon au moins l'une des revendications 1 à 3, caractérisé en ce que le bain d'agent nettoyant présente après la neutralisation ou l'alcalisation une valeur de pH comprise entre 7 et 12, en particulier entre 9 et 12.

5. Procédé selon au moins l'une des revendications 1 à 4, caractérisé en ce que la désinfection thermique s'effectue à des températures supérieures à 84°C, en particulier supérieures à 90°C.

6. Procédé selon la revendication 5, caractérisé en ce que la désinfection thermique s'effectue pendant au moins 3 minutes, en particulier au moins 10 minutes.
